# EUROPÄISCHE PATENTSCHRIFT

(11) **EP 3 540 044 B1**
(45) Veröffentlichungstag und Bekanntmachung des Hinweises auf die Patenterteilung: **07.06.2023**
(21) Anmeldenummer: 18161443.9
(22) Anmeldetag: 13.03.2018
(51) Int. Cl.: C12N 1/22, C12N 9/24, C12N 9/42, C12P 19/14, C12P 7/06

(54) **VERFAHREN ZUR HERSTELLUNG VON CELLULOLYTISCHEN ENZYMEN IN XYLOSE-HALTIGEN HYDROLYSATEN ZUM ABBAU VON LIGNOCELLULOSEHALTIGER BIOMASSE**
PROCESS FOR THE PREPARATION OF CELLULOLYTIC ENZYMES IN XYLOSE-CONTAINING HYDROLYSATES FOR THE DEGRADATION OF A BIOMASS CONTAINING LIGNOCELLULOSE
PROCÉDÉ DE PRODUCTION D'ENZYMES CELLULOLYTIQUES DANS DES HYDROLYSATS CONTENANT DU XYLOSE DESTINÉ À LA DÉGRADATION DE LA BIOMASSE LIGNOCELLULOSIQUE

(43) Veröffentlichungstag der Anmeldung: 18.09.2019
(73) Patentinhaber: Prüf- und Forschungsinstitut Pirmasens e.V., 66953 Pirmasens (DE)
(72) Erfinder: BALLMANN, Patrick, 66953 Pirmasens (DE); DRÖGE, Stefan, 55232 Alzey (DE); MÜLLER, Michael, 53332 Bornhei-Waldorf (DE)
(74) Vertreter: Patentanwälte Dr. Keller, Schwertfeger Partnerschaft mbB

(56) Entgegenhaltungen:
- WO-A1-2017/177289
- MÜLLER MICHAEL ET AL: "Continuous xylanase production with Aspergillus nidulans under pyridoxine limitation using a trickle bed reactor", BIORESOURCE TECHNOLOGY, ELSEVIER, AMSTERDAM, NL, Bd. 188, 2. Februar 2015 (2015-02-02), Seiten 219-225, XP029156445, ISSN: 0960-8524, DOI: 10.1016/J.BIORTECH.2015.01.085
- OLIVEIRA GORGULHO SILVA CAIO DE ET AL: "A Review of Holocellulase Production Using Pretreated Lignocellulosic Substrates", BIOENERGY RESEARCH, SPRINGER US, BOSTON, Bd. 10, Nr. 2, 7. Februar 2017 (2017-02-07), Seiten 592-602, XP036229262, ISSN: 1939-1234, DOI: 10.1007/S12155-017-9815-X [gefunden am 2017-02-07]

## Beschreibung

Die vorliegende Erfindung betrifft Verfahren zur Herstellung von cellulolytischen Enzymen mit filamentösen Pilzen in xylosehaltigen Substraten zum Abbau von lignocellulosehaltigen Biomassen nach einer thermisch-chemischen Vorbehandlung. Die Expression der Enzyme erfolgt durch die im Medium enthaltene Xylose in einem wachstumslimitierenden Prozess in einem Rieselstromreaktor. Beschrieben sind auch Verfahren zur Herstellung von Zuckern, Biokraftstoffen, Biokunststoffen oder pharmazeutischen/chemischen Produkten auf Basis eines erfindungsgemäßen Verfahrens.

Die zunehmenden Probleme im Bereich Klima und Umwelt durch den massiven Einsatz von fossilen Rohstoffen machen Alternativen notwendig. Biomassen auf Basis von Lignocellulose machen den Großteil der verfügbaren nachwachsenden Rohstoffe auf der Erde aus und stehen somit als potentielle Alternative zu fossilen Rohstoffen wie Erdöl oder Erdgas für eine energetische und stoffliche Nutzung zur Verfügung. Hauptbestandteile der Lignocellulose sind die drei Fraktionen Cellulose, Hemicellulose und Lignin. Den prozentual größten Anteil macht hierbei die Cellulose aus. Das Polysaccharid Cellulose besteht aus langen Ketten von mehreren hundert bis tausend unverzweigten β (1→4)-D-Glucose-Einheiten. Cellulose ist ein wichtiger struktureller Bestandteil der primären Zellwände in Pflanzen. Hemicellulose ist ein Heteropolymer, welches Xylan, Glucoronoxylan, Arabinoxylan, Glucomannan und Xyloglucan umfasst. Etwa 80 % der Hemicellulose besteht aus Xylan, eine Verbindung aus Xylose-Monomeren. Hemicellulose enthält darüber hinaus weitere Zucker wie Xylose, Arabinose, Glucose, Mannose, Galactose und Rhamnose. Im Vergleich zu Cellulose sind die Ketten der Hemicellulose erheblich kürzer und verzweigter.

Lignin ist ein komplexes organisches Polymer, bestehend aus unterschiedlichen phenolischen Verbindungen, und spielt bei der mechanischen Stabilität der Pflanzen eine wichtige Rolle. Es lagert sich zwischen die Cellulose und Hemicellulose ein. Die genaue Zusammensetzung der Lignocellulose hängt von verschiedenen Faktoren ab wie z.B. den Wachstumsbedingungen oder dem Standort der Pflanze (Howard, Abotsi et al. 2004). Beim Weizenstroh z.B. kann der Celluloseanteil zwischen 27 - 38 %, der Hemicelluloseanteil zwischen 16 - 26 % und der Ligninanteil zwischen 15- 22 % variieren.

Eine effektive energetische und stoffliche Nutzung der Lignocellulose setzt eine Umwandlung der beiden Hauptpolymere Cellulose und Hemicellulose in fermentierbare Zucker voraus. Die wichtigsten Zucker sind hierbei die Glucose und Xylose. Hierfür stehen verschiedene biologische, physikalische, chemische und Kombinationen von Verfahren zur Verfügung. Ein State-of-the Art Verfahren ist die thermische Vorbehandlung der Biomasse bei Temperaturen zwischen 140 - 200 °C in wässriger Umgebung (Chen, Liu et al. 2017). Um den Abbau der Lignocellulose zu beschleunigen, werden in geringen Mengen Säuren oder Laugen zugegeben. Die amorphen Strukturen der Hemicellulose begünstigen einen Abbau, so dass sich die enthaltenen Zucker, hauptsächlich Xylose und Arabinose in der flüssigen Phase, im Folgenden als Hydrolysat bezeichnet, lösen. Die kristallinen Strukturen der Cellulose sind deutlich widerstandsfähiger, ein effektiver Abbau benötigt daher deutlich höhere Temperaturen von über 220 °C oder höhere Säure-Konzentrationen (Hall, Bansal et al. 2010). Dieses hat allerdings einen hohen Energie- bzw. Chemikalienverbrauch zur Folge. Eine bedeutende Alternative zum thermischen Cellulose-Abbau ist eine enzymatische Umsetzung mit cellulolytischen Enzymen (Alvira, Tomäs-Pejö et al. 2010). Die enzymatische Umsetzung erfordert unterschiedliche cellulolytische Aktivitäten, wie eine Cellobiohydrolase, eine Endoglucanase und eine β-Glucosidase. Die Cellobiohydrolase spaltet die Cellulose-Kette in kleinere Cellobioseeinheiten, bestehend aus zwei Glucose-Monomeren, ausgehend von den Kettenenden. Die Endoglucanase führt eine Spaltung innerhalb der Cellulose-Ketten durch, wodurch sich die Kettenlänge verringert und wiederum eine höhere Anzahl an freien Enden für die Cellobiohydrolase geschaffen wird. Die β-Glucosidase-Aktivität spaltet die Cellobiose-Einheiten in Glucose-Monomere (Gottschalk, Oliveira et al. 2010). Als Endprodukt steht eine konzentrierte Zuckerlösung mit einer hohen Glucose-Konzentration zur Verfügung. Die Zucker-Lösung kann in bekannten Fermentationsprozessen weiterverarbeitet werden, beispielsweise zur Herstellung von Biokraftstoffen, Biokunststoffen oder anderen pharmazeutischen und/oder chemischen Produkten (Menon and Rao 2012).

Durch die steigende Nutzung von Lignocellulose als nachwachsender Rohstoff ist der Bedarf an cellulolytischen Enzymen hoch, weshalb eine kosteneffektive Enzymproduktion notwendig ist. Für die Produktion großer Mengen an cellulolytischen Enzymen sind drei verschiedene Faktoren essentiell: ein Produktionsstamm mit hohen Expressions- und Sekretionsraten, günstige Substrate für die Enzymproduktion und ein effektives Produktionsverfahren. Die zum enzymatischen Abbau benötigten Enzyme können auf natürliche Weise durch verschiedene Mikroorganismen gewonnen werden. Hierbei spielen filamentöse Pilze eine sehr wichtige Rolle. Vor allem die Gattungen *Trichoderma* und *Aspergillus* setzen große Mengen an cellulolytischen Enzymen frei. Verschiedene Arten werden bereits für eine biotechnologische Produktion eingesetzt (Gottschalk, Oliveira et al. 2010, Jun, Kieselbach et al. 2011, Zoglowek, Lübeck et al. 2015). Als Produktionsmedium können verschiedene Substrate eingesetzt werden, Avicel oder kristalline Cellulose eigenen sich besonders gut, sind aber auch sehr teuer. Daher wurden in den letzten Jahren umfangreiche Untersuchungen mit landwirtschaftlichen Resten als Substrat für die Enzymproduktion durchgeführt (Jecu 2000, da Silva Delabona, Pirota et al. 2012, Lee, Lee et al. 2017). Allerdings müssen diese Substrate in separaten Prozessen anfallen und dem Prozess der Enzymproduktion extra zu geführt werden. Eine Möglichkeit wäre es auf einem Teil lignocellulosehaltigen Biomasse, welche im Anschluss verzuckert werden soll, die benötigten Enzyme zu produzieren. So existieren Verfahren in den z.B. Stroh als Kohlenstoffquelle genutzt werden, allerdings steht in diesem Falle das Stroh nicht mehr vollständig zur anschließenden Verzuckerung zur Verfügung (Kogo, Yoshida et al. 2017). Neben festen Substraten existieren auch Verfahren mit flüssigen Substraten wie z.B. Lactose (Xu, Gao et al. 2017). Allerdings muss diese auch extern dem Prozess zugeführt werden, was wiederum die Produktionskosten erhöht. Der dritte entscheidende Faktor ist das Produktionsverfahren. Ein effektives Produktionsverfahren hat die Aufgabe, große Mengen an Enzym bereitzustellen und diese gleichzeitig in einem Downstream-Verfahren einfach zur Verfügung zu stellen. Hierbei werden die oben genannten landwirtschaftlichen Reststoffe häufig in einem Solid-State-Verfahren (SSF) eingesetzt, um cellulolytische Enzyme zu produzieren (Lee, Lee et al. 2017, Soccol, da Costa et al. 2017). Ein Verfahren zur simultanen Herstellung von cellulolytischen Enzymen und Biomasse-Hydrolyse durch genetisch modifizierte Pilzstämme wie zum Beispiel *Trichoderma reesei* wurde unter Verwendung von xylosehaltigem Hydrolysat angewandt (WO 2017/177289 A1, 2017). Filamentöse Pilze wachsen hierbei ähnlich wie in ihrer natürlichen Umgebung als dichtes Pilzmycel. Im Vergleich zu konventionellen Submers-Fermentationen (SmF) sind keine schädlichen Scherkräfte vorhanden. Außerdem ist der Wasser- und Energieverbrauch deutlich geringer. Allerdings ist die Versorgung mit Sauerstoff und Nährstoffen deutlich schlechter. Für eine weitreichende Produktion im SSF-Verfahren ist eine große Fläche notwendig. Außerdem müssen beim Downstream-Prozess größere Mengen Flüssigkeit eingesetzt werden, um die Enzyme auszuwaschen. Dies reduziert wiederum die Energie- und Wasserersparnis. Außerdem wird die Enzym-Lösung verdünnt. Im SmF-Verfahren ist die Versorgung mit Sauerstoff-Nährstoffen besser. Auch der Flächenverbrauch ist geringer, allerdings sind hier die Scherkräfte höher, was zu Verlusten bei den Enzymausbeuten führen kann (Ghobadi, Ogino et al. 2017) . Außerdem muss beim Downstream-Prozess die Biomasse von der flüssigen Enzym-Lösung getrennt werden, was wiederum Energie kostet. Außerdem eignen sich viele landwirtschaftliche Reststoffe nicht für eine Flüssigkultivierung.

Im Rieselstromreaktor kann Xylanase mittels *Aspergillus nidulans* produziert werden. Hierfür wurde der Stamm mit einem Pyridoxin(pyroA4)-Marker versehen und genetisch modifiziert zur Überexpression von Xylanase B (Müller, Prade et al. 2015). Allerdings kann diese genetische Modifikation nicht genutzt werden, um Cellulasen zu produzieren.

Vor diesem Hintergrund ist es Aufgabe der vorliegenden Erfindung, ein alternatives und zugleich verbessertes Herstellungsverfahren für die Produktion von cellulolytischen Enzymen wie Cellobiohydrolasen, Endoglucanasen und β-Glucosidasen mit filamentösen Pilzen in einem günstigen Produktionsmedium mittels eines Bioreaktors bereitzustellen.

Diese Aufgabe wird gelöst durch ein Verfahren zur Herstellung von cellulolytischen Enzymen mit den Merkmalen des Anspruchs 1. Bevorzugte Ausführungsvarianten sind Bestandteil der Unteransprüche.

Das erfindungsgemäße Verfahren ermöglicht die großtechnische Herstellung cellulolytischer Enzyme mit Hilfe genetisch modifizierter filamentöser Pilzstämme unter Einsatz von xylosehaltigen Hydrolysaten aus der thermisch-chemisch Vorbehandlung von Lignocellulose. Um lignocellulosehaltige Biomassen effektiv verzuckern zu können ist eine Vorbehandlung notwendig. Hierbei fallen größere Mengen an xylosehaltigen Hydrolysaten an. Die im Verfahren eingesetzte lignocellulosehaltige Biomasse kann beispielsweise aus Kulturpflanzen wie z.B. Hirse, Pappelholz, Landwirtschaftsresten (z.B. Stroh, Reisschalen, Getreideresten) und Resten aus der Forstwirtschaft stammen (z.B. Holz, Zweige etc.). Durch die Nutzung der anfallenden Hydrolysate steht ein günstiges Substrat zur Verfügung. Die Feststofffraktion kann somit vollständig einer Verzuckerung zugeführt werden. Die Produktion der erwünschten Cellulasen wie Cellobiohydrolasen, Endoglucanasen, und β-Glucosidasen erfolgt im Hydrolysat, welches den Induktor enthält, also vorzugsweise Xylose oder Xylan. Hierzu werden die natürlichen Promotoren der Zielgene durch einen eigenen Xylanase-Promotor ersetzt. Um zusätzlich die Enzymsekretion zu erhöhen, wird ein spezifischer Transkriptionsaktivator überexprimiert, welcher natürlicherweise die Expression der hemicellulolytischen Enzyme wie Xylanasen reguliert. Durch die Nutzung eines Xylanase-Promotors für die Expression der cellulolytischen Enzyme, wird durch die Deregulierung des Transkriptionsaktivators auch die Expression der cellulolytischen Enzyme in den neu geschaffenen Stämmen erhöht. Vorzugsweise werden die erfindungsgemäßen genetisch modifizierten filamentösen Pilzstämme in einem Rieselstromreaktor unter wachstumslimitierenden Bedingungen kultiviert, um eine möglichst große Ausbeute an cellulolytischen Enzymen zu erreichen. Der Stamm wächst hierbei auf Kunststofffüllkörpern in einem Zwei-Stufen-Prozess. In der ersten Phase wächst der Pilz unter nicht-limitierenden Bedingungen im Hydrolysat zur Bildung von ausreichend Biomasse. Während der zweiten Stufen werden wachstumslimitierende Bedingungen eingestellt. Die künstlich hervorgerufenen wachstumslimitierenden Bedingungen werden beispielsweise durch Entzug eines Wachstumsfaktors geschaffen. Dadurch produzieren die Pilzstämme nach wie vor die exprimierten Enzyme, ein weiteres Wachstum oder Vermehrung der Pilze bleibt jedoch überwiegend aus.

Erfindungsgemäß wird in einem ersten Verfahrensschritt zur Herstellung von cellulolytischen Enzymen ein genetisch modifizierter filamentöser Pilzstamm mit der Fähigkeit zur Produktion von Cellobiohydrolasen, Endoglucanasen, Exocellulasen und/oder β-Glucosidasen über ein Genkonstrukt bereitgestellt, beim dem ein in das Genkonstrukt klonierter Transkriptionsfaktor überexprimiert wird. Bei dem Transkriptionsaktivator handelt es sich vorzugsweise um XInR von *Aspergillus nidulans* oder Xyr1 von *Trichoderma reseei.* Aber auch gleichartige Transkriptionsaktivatoren in anderen filamentösen Pilzen können im Sinne der vorliegenden Erfindung benutzt werden. Die Überexpression erfolgt über das Ersetzen des natürlichen Promotors durch einen konstitutiv stark exprimierenden Promoter, wie beispielsweise den konstitutiven Promotor gpdA, welcher die Glyceraldehyd-3-Phosphatdehydrogenase reguliert. Hierdurch erfolgt eine Überexpression der Xylanase-Produktion. Der neu geschaffene Stamm wird in einem xylosehaltigen Stamm kultiviert. Filamentöse Pilze wie A. *nidulans* setzen in der Regel verschiedene Xylanasen frei. Die stärkste Xylanase-Aktivität wird bestimmt und die dazu gehörige Promotorsequenz ausgewählt. Für die Produktion der cellulolytischen Enzyme werden die natürlichen Promotorsequenzen durch den ausgewählten Xylanase-Promotor ausgetauscht. Die neu geschaffenen Gensequenzen werden in den Stamm mit dem deregulierten Transkriptionsaktivator kloniert.

In einem weiteren Verfahrensschritt erfolgt eine Kultivierung des genetisch modifizierten filamentösen Pilzsstammes in einem Xylose- oder Xylan-haltigen Hydrolysat, welches bevorzugt zuvor durch eine thermisch-chemische Vorbehandlung von lignocellulosehaltiger Biomasse zur Verfügung gestellt wurde. Anschließend erfolgt eine Isolierung der so produzierten celluloytischen Enzyme.

Erfindungsgemäß wird das Xylose- oder Xylan-haltige Hydrolysat aus einem Vorbehandlungsprozess gewonnen, bei dem der Hemicellulose-Anteil der lignocellulosehaltigen Biomasse aufgespalten wird. Die infolge des Vorbehandlungsprozesses erhaltene flüssige Fraktion (Hydrolysat) enthält eine große Menge an Xylose und Arabinose. Als lignocellulosehaltige Biomasse kommt vorzugsweise Weizen-, Roggen-, Mais- oder Reisstroh zum Einsatz. Vorzugsweise wird hierzu in einem vorgeschalteten Verfahrensschritt die Lignocellulose thermisch-chemisch vorbehandelt, um den Komplex zwischen Hemicellulose und Cellulose aufzubrechen. In einer bevorzugten Variante wird für die Aufspaltung von Lignocellulose eine thermale Hydrolyse unter Anwendung gelöster Mineralsäuren, wie Salpetersäure oder Schwefelsäure, verwendet. Während dieser Vorbehandlung wird der überwiegende Anteil an Hemicellulose in Xylose und Arabinose gespalten, was bedingt ist durch die amorphe Struktur von Hemicellulose, welche gegenüber einer sauren Hydrolyse-Behandlung besonders empfindlich ist. Die freigesetzte Xylose oder Xylane dient als Induktor für eine Expression dieser cellulolytischen Enzyme. Durch die erfindungsgemäße Bereitstellung von Xylose in dem Hydrolysat wird eine kostengünstige Alternative zu käuflicher Xylose als Substrat bereitgestellt. Aufgrund der kristallinen Struktur der Polysaccharide verbleibt der Cellulose-Anteil nahezu vollständig in der Feststofffraktion. Das beschriebene Verfahren ermöglicht eine kostengünstige Herstellung cellulolytischer Enzyme, welche wiederum entsprechend dem Kreislaufprinzip zum Abbau des Cellulose-Anteils verwendet werden kann.

Die Herstellung der Enzyme kann auf unterschiedliche Art und Weise erfolgen. Üblicherweise wird zwischen einer Solid-State-Fermentation (SSF) und Submer-Fermentation (SmF) unterschieden. Beim SSF-Prozess wächst der Pilz als Mycel auf einer festen Oberfläche, was die Enzymproduktion begünstigt. Die Wasser- und Energienutzung während der Kultivierung ist relativ gering. Allerdings sind die nachfolgenden Prozessschritte sehr kompliziert, da das freigesetzte Enzym zunächst mit reichlich Wasser ausgewaschen werden muss, wodurch die Enzymkonzentration nur gering ist. Auch das Vermengen und die Bereitstellung von Sauerstoff oder Nährstoffen sind schwierig. Beim SmF-Prozess sind die Vermischung und die Sauerstoffbereitstellung verbessert. Allerdings sind die Scherkräfte deutlich höher, was wiederum zu einem pelletartigen Pilzwachstum führt. Dies kann unter Umständen die Enzymproduktion senken. Außerdem muss am Ende des Prozesses die Biomasse von der Flüssigkeit durch einen zusätzlichen Produktionsschritt getrennt werden. Dies erhöht wiederum die Gesamtkosten für die Enzym-Produktion.

Das erfindungsgemäß zum Einsatz kommende Rieselstromverfahren verbindet nun die Vorteile beider Prozesse in einem Festbettreaktor. Der genetisch modifizierte Pilzstamm wächst als Mycel auf der Oberfläche in dem Reaktor, wobei vorzugsweise Festkörper mit Oberflächen zum Einsatz kommen, die von dem Pilz bewachsbar sind. Das Hydrolysat wird von der Kopfseite des Reaktors beaufschlagt, welches als Flüssigkeit so von oben nach unten rinnt und über eine Pumpe wiederum nach oben befördert wird. Die Sauerstoffversorgung erfolgt über ein sauerstoffhaltiges Gasgemisch (vorzugsweise Luft) von der Unterseite des Reaktors. Die Luft strömt dann von unten nach oben durch das Reaktorinnere im Gegenstrom über Flüssigkeit des Hydrolysats. Ähnlich dem SSF-Prozess können die genetisch modifizierten Pilzstämme in ihrer natürlichen Form als Mycel auf den Festkörperoberflächen wachsen, während die hergestellten Enzyme in das Hydrolysat entlassen werden. Dadurch erfolgt eine Aufkonzentrierung der cellulytischen Enzyme in dem Hydrolysat. Im Gegensatz zum SSF-Verfahren aber kann eine ausreichende Sauerstoff- und Nährstoffversorgung zur Verfügung gestellt werden, ohne dass es zu erhöhten Scherkräften während des Prozesses kommt. Das Hydrolysat mit den Enzymen kann ohne Filtration oder Zentrifugation über einen am Reaktor angebrachten Ablauf entnommen werden. Somit ist der Downstream-Prozess viel einfacher, da die Enzym-Lösung nicht von der Biomasse abgetrennt werden muss und auch keine Verdünnung durch Zugabe von weiterer Flüssigkeit erfolgt.

Um ein Zuwachsen des Reaktors durch sich ständig vermehrende Pilze zu vermeiden, ist in einer bevorzugten Ausführungsform vorgesehen, dass der genetisch modifizierte filamentöse Pilzstamm eine auxotrophe Mutante ist, die zum Wachstum einen spezifischen Wachstumsfaktor benötigt. Vorzugsweise handelt es sich bei dem Wachstumsfaktor um p-Aminobenzoesäure oder Pyroxidin. Fehlt der Wachstumsfaktor, unterbleibt auch ein Wachstum der Pilze, allerdings ohne größere Auswirkungen auf die erwünschte Enzymproduktion. Somit kann dem System der Wachstumsfaktor entzogen werden, sobald die Festkörper ausreichend mit Pilzmycel überlagert sind, so dass in der nachfolgenden zweiten Phase überwiegend die Enzymproduktion der cellulolytischen Enzyme stattfindet. In der ersten Phase erfolgt somit das Pilzwachstum zum Aufbau von Biomasse, während die Enzymherstellung in der zweiten Phase ohne weitere Biomassenvermehrung weiterläuft. Ein besonderer Vorteil des Verfahrens ist darin zu sehen, dass das Hydrolysat in der zweiten Phase keine nennenswerten Mengen an Wachstumsfaktor enthält.

Das aus der Vorbehandlung der lignocellulosehaltigen Biomasse stammende Hydrolysat wird in einer bevorzugten Variante vorzugsweise mit einem Mineralmedium vermengt. Vorzugsweise enthält das Mineralmedium Natriumnitrat, Kaliumchlorid, Magnesiumsulfat, Kaliumdihydrogenphosphat und/oder Spurenelemente. In der Wachstumsphase enthält das Hydrolysat zusätzlich Wachstumsfaktoren, beispielsweise p-Aminobenzoesäure (PABA) oder Pyroxidin.

Das erfindungsgemäße Verfahren ermöglicht nicht nur die Herstellung von cellulolytischen Enzymen, sondern in nachfolgenden Schritten auch die Herstellung von Zuckern (Glucose) durch Inkubation der im Prozess erhaltenen celllulolytischen Enzyme mit thermisch vorbehandelter lignocellulosehaltiger Biomasse. Durch die cellulolytische Enzymaktivität wird Lignocellulose letztendlich zu Zucker abgebaut, welcher wiederum zu anderen Verfahrenszwecken eingesetzt werden kann. So kann das erfindungsgemäße Verfahren zur Herstellung von Biokraftstoffen, Biokunststoffen oder pharmazeutischen und/oder chemischen Produkten verwendet werden, indem die über das erfindungsgemäße Verfahren erhaltenen cellulolytischen Enzyme mit thermisch vorbehandelter lignocellulosehaltiger Biomasse zum Erhalt einer Zuckerlösung inkubiert werden und die so erhaltene Zuckerlösung anschließend mit Mikroorganismen zu den jeweiligen Produkten weiterverarbeitet wird, beispielsweise im Zuge einer Fermentation. Die bei der enzymatischen Hydrolyse anfallenden Reste können ferner zur Gewinnung von Biogas ausgenutzt werden.

Die Erfindung wird in den nachfolgenden Ausführungsbeispielen näher erläutert.

### Thermische Vorbehandlung der lignocellulosehaltigen Biomasse

Die Vorbehandlung der lignocellulosehaltigen Biomasse (Stroh) diente zur Herstellung des Ausgangs-Hydrolysats und wurde mit Hilfe einer thermischen Druckhydrolyse unter Zusatz von verdünnter Salpetersäure durchgeführt. Die Endkonzentration der Salpetersäure im Wasser lag bei 0.45 % (v/v). Die Konzentration der Salpetersäure hatte Einfluss auf den Aufschlussgrad der Biomasse. Geringere Säurekonzentrationen führten zu einer verminderten Xylose-Konzentration. Höhere Konzentrationen können allerdings das Reaktorgefäß beschädigen. Das Strohgemisch wurde mit der verdünnten Säure bis zu einem Trockengehalt von 15 % (v/w) vermengt. Vor der Behandlung wurde die Zusammensetzung der lignocellulosehaltigen Biomasse (Stroh) nach einem Standardverfahren des "National Renewable Energy Laboratory" (NREL, Colorado, USA)) bestimmt (Sluiter, Hames et al. 2008). Die Behandlung der Biomasse erfolgte bei einer Temperatur zwischen 140 und 160° C, mit einer Behandlungszeit zwischen 15 und 60 Minuten. Der Reaktor wurde anschließend in einem ersten Schritt auf eine Temperatur von 120° C erhitzt.

In einem zweiten Schritt erfolgte der Aufheizprozess auf die gewünschte Behandlungstemperatur. Der Aufheizprozess erfolgte über die Einleitung von Heißdampf. Nach Erreichen der Behandlungstemperatur wurde die Behandlung gestartet. Im Anschluss an die Behandlung wurde der Reaktor abgekühlt und es erfolgte ein schneller Druckablass bei 2 bar. Anschließend wurden die flüssigen und die festen Fraktionen voneinander getrennt, das Volumen des Hydrolysats und das Gewicht der Festfraktion bestimmt. Die Konzentrationen der Zucker und Abbauprodukte im Hydrolysats wurden durch eine Analyse mittels HPLC bestimmt. Der Hauptbestandteil des Hydrolysats war Xylose mit Konzentrationen zwischen 9 - 11 g/l. Daneben enthielt das Hydrolysat auch Arabinose und Glucose mit Konzentrationen zwischen 3 - 5 g/l. Die Konzentration der Zucker hängt von den jeweiligen Vorbehandlungsbedingungen ab. Bei höheren Temperaturen oder längeren Behandlungszeiten steigt in der Regel die Menge an freigesetzten Zuckern. Allerdings beeinflussen die Vorbehandlungsbedingungen auch die Bildung von Nebenprodukten. Hohe Temperaturen und lange Behandlungszeiten erhöhen den Abbau von Lignin zu verschiedenen phenolischen Nebenprodukten. Außerdem können freigesetzte Zucker, wie Xylose oder Glucose weiter zersetzt werden zu Furfural oder 5-HMF bzw. Ameisensäure und CO₂. Ein solcher Abbau reduziert die erzielten Ausbeuten an Zuckern. Die Hydrolysate enthielten auch organischen Säuren wie z.B. Essigsäure oder Ameisensäure, die zum Teil bei der Zersetzung von Hemicellulose freigesetzt wurden. Um die Bildung von Nebenprodukten zu reduzieren, sollte die Behandlungstemperatur und -zeit nicht über 160 °C sein. Die Festfraktion wurde bei einer Temperatur von 50° C getrocknet und anschließend einer Strukturanalyse entsprechend der Verfahren der NREL (National Renewable Energy Laboratory, USA) unterzogen. Die flüssige Fraktion (Hydrolysat) wurde zur Enzymproduktion verwendet. Um die Zucker-Konzentrationen im Hydrolysat zu erhöhen, wurde das Hydrolysat mittels eines Fallstromverdampfers aufkonzentriert. Hohe Zucker-Konzentrationen ermöglichen eine stärkere Enzym-Produktion. Die Endkonzentration an Xylose lag zwischen 160 - 180 g/l. Durch den Aufkonzentrierungsprozess wurden zusätzlich flüchtige Bestandteile wie das Furfural, welches in größeren Mengen eine hemmende Wirkung auf Mikroorgansimen hat, aus dem Hydrolysat entfernt. Außerdem sank der pH-Wert im Hydrolysat, wodurch die Lieferfähigkeit verbessert wird, insbesondere weil die Gefahr einer Fremdkontamination reduziert wird. Die Festfraktion aus der Vorbehandlung konnte für die enzymatische Hydrolyse verwendet werden, um fermentierbare Glucose-Lösungen zu erhalten.

### Herstellung von genetisch modifizierten Xylose- oder Xylan-induzierbaren filamentösen Pilzstämmen

Als Modellorganismus wurde stellvertretend für *Aspergillus nidulans, Aspergillus niger, Aspergillus vadensis, Aspergillus feotidus, Aspergillus oryzae, Aspergillus aculeatus, Trichoderma reesei, Trichoderma harzianum, Neurospora crassa* der Pilzstamm *Aspergillus nidulans A773 (pyrG89; wA3; pyroA4)* vom Fungal Genetics Stock Center (FGSC, St. Louis, USA) bezogen. Es wurden unterschiedliche genetische Modifikationen durchgeführt. Zum einen wurde eine Mutante mit einem überexprimierbaren XInR-Transkriptionsaktivator erzeugt. Hierzu wurde ein gpdAP-gesteuertes XLnRA-Überexpressionskonstrukt in den pabaA Lokus des Chromosoms I von *A*. *nidulans* kloniert. Die Modifikation erfolgte entsprechend der Gibson-Methode. Der Klonierungserfolg wurde durch PCR-Amplifikation validiert.

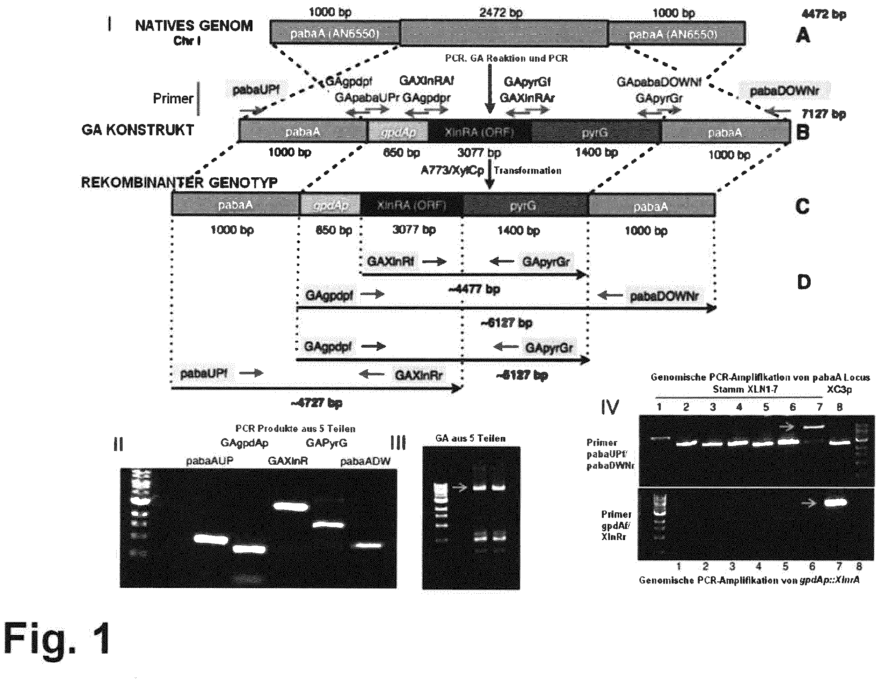

In Fig. 1 ist der Aufbau des gpdAP-gesteuerten XInRA-Überexpressionskonstrukts gezeigt, dass in den pabaA-Lokus des Chromosoms 1 kloniert wurde. A zeigt den nativen pabaA (AN6550) Locus auf Chromosom I. B bezeichnet das GA (Gibson Assembly)-Konstrukt mit den fusionierten PCR-Fragmenten. In C ist der rekombinante Geno-Typ nach einer homologen Rekombination (doppelte Crossover-Integration) des GA-Konstruktes in das *Aspergillus nidulans-Genom* gezeigt. D zeigt die Auswahl der PCR-Primer und deren erwartete Produkte, die zur Validierung des neu erzeugten Locus eingesetzt wurden. II zeigt die amplifizierten DNA-Stücke der Produkte und III die entsprechenden Kontrollen. IV zeigt die Validierung unterschiedlicher *A*. *nidulans* (A773) Transformanten, die das korrekte gpdAP::xInRA-Konstrukt in den pabaA-Lokus integriert haben.

Eine Mutante (Fig. 1, IV, Spur 7, Pfeil) hatte die korrekte Modifikation und wurde für die weiteren Tests verwendet. Diese Mutante führte zu einer Überexpression des XInRA-Gens und der Stamm wuchs in Abhängigkeit von p-Aminobenzoesäure (paba) und Pyridoxin. Der so erhaltene neue Stamm (PFI-xyl) wurde in einem Medium mit 2 % Xylose getestet. Die in das Medium ausgesonderte hauptsächliche Hauptxylanase war Xylanase C (Endo-1,4-β-Xylanase).

Beim nächsten Klonierungsschritt wurden die Modifikationen durchgeführt, welche die Expression des erwünschten cellulolytischen Enzyms (Endoglucanase, Exocellulase, Cellobiohydrolase, β-Glucosidase) durch Xylose oder Xylan ermöglichen. Beispielhaft soll dies anhand von drei unterschiedlichen Stämmen gezeigt werden, die jeweils drei Einzelaktivitäten aufweisen. Dazu wurden die Gene für Endoglucanase, Exocellulase und β-Glucosidase zusammen mit einem xynC-Promotor (Promotor der Xylanase C) zufällig in den PFI-xyl-Stamm integriert. Die Auswahl des Xylanase-Promotors erfolgte basierend auf der Expression eines Stammes, der in dem Hydrolysat mit Xylose kultiviert wurde. Für die Erfindung kam der stärkste Xylanase-Promotor für die Expression der cellulolytischen Enzyme zum Einsatz. Die daraus resultierenden Stämme wurden ebenfalls in Xylose getestet. In allen Stämmen wurde die Expression der cellulolytischen Enzyme durch Xylose induziert. Jeder einzelne Stamm wies eine eigene cellulolytische Aktivität auf: PFI-egl (Endoglucanase-Aktivität), PFI-exo (Exocellulase-Aktvität) und PFI-bgl (β-Glucosidase-Aktivität). Von der Erfindung umfasst sind jedoch auch Stämme, bei denen auch mehr als eine Enzymaktivität, beispielsweise alle erwünschten cellulolytischen Enzymaktivitäten, in das Genom des Pilzstammes integriert sind. Somit kann eine Co-Besiedelung des Reaktors mit einem oder mehreren Pilzstämmen, entsprechend der erwünschten Enzymaktivität, erfolgen. Die Expression der cellulolytischen Enzyme in *A*. *nidulans* konnte durch Xylan oder Xylose induziert werden. Auf diese Weise konnten alle vier Xylanase-Aktivitäten von *Aspergillus nidulans* stimuliert werden.

Bei den Trichodermastämmen wurde der Transkriptionsaktivator Xyr1 verwendet, welcher in ähnlicher Weise die Expression der cellulolytischen und xylanolytischen Gene reguliert. Gleichermaßen führt auch hier der Austausch des natürlichen Promotors der Gene für die cellulolytischen Enzyme durch einen Xylase- oder Xylan-induzierbaren Promotor zu einer vermehrten Expression der cellulolytischen Enzyme, die sich großtechnisch weiterverarbeiten lassen.

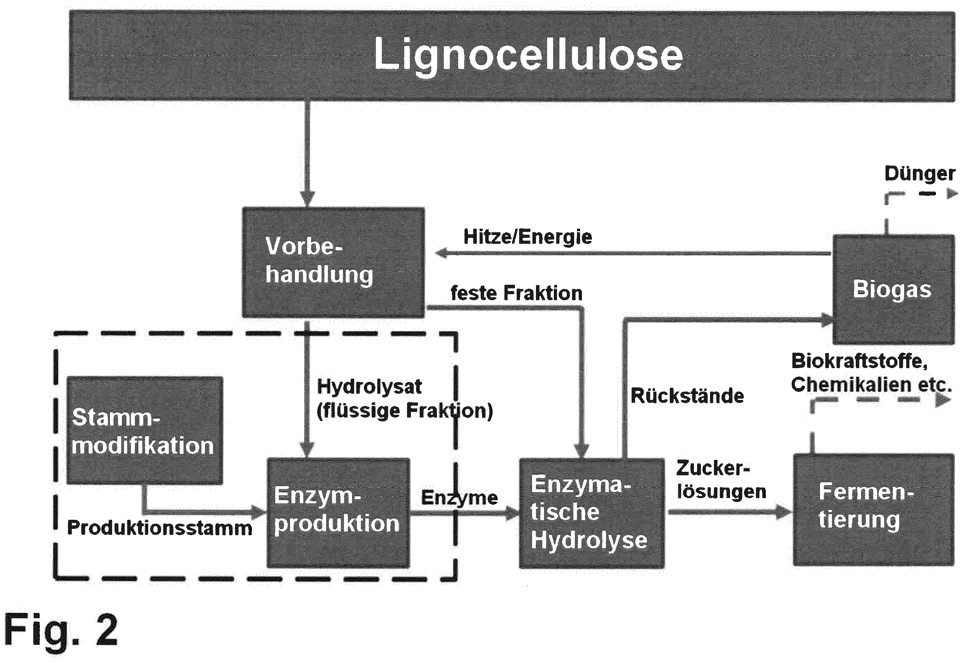

In Fig. 2 ist ein Schema über den Verfahrensablauf zu sehen. Ausgangspunkt ist eine thermische Vorbehandlung von Lignocellulose (z.B. Stroh oder anderen lingocellulosehaltigen Biomassen), die vorzugsweise unter Einsatz von gelösten Säuren durchgeführt wird. Bei der thermischen Vorbehandlung der lignocellulosehaltigen Biomasse entsteht ein Hydrolysat als flüssige Fraktion, welches zur Enzymherstellung im Rieselstromverfahren eingesetzt wird. Vorzugsweise erfolgt bei diesem Schritt die Zugabe eines Wachstumsfaktors für den verwendeten genetisch modifizierten filamentösen Pilzstamm. Deshalb wird vorzugsweise eine auxotrophe Mutante verwendet, bei der das Wachstum ohne Anwesenheit des spezifischen Wachstumsfaktors (oder eines Gemisches von Wachstumsfaktoren) zum Erliegen kommt. Das Hydrolysat enthält eine große Menge an Xylose oder Xylan, welches wiederum zu einer Expression von den Cellobiohydrolasen, Endoglucanasen, Exocellulasen und/oder β-Glucosidasen führt. Hierbei kommt ein Rieselstromreaktor zum Einsatz, bei dem die Pilze auf Kunststoffkörpern als Mycel wachsen. Die Modifikation der Pilzstämme erfolgte zum einen durch Austausch des natürlichen Promotors durch einen Xylanase-Promotor und durch eine Überexpression eines Transkriptionsaktivators welcher natürlicherweise die Expression der Xylanase-Gene steuert. Cellulolytische Gene welche ebenfalls unter der Kontrolle von Xylanase-Promotoren standen wurden somit ebenfalls überexprimiert.

Die bei der thermischen Vorbehandlung entstehende Festfraktion kann einer enzymatischen Hydrolyse zugeführt werden. Auf diese Weise kann Zucker (Glucose) hergestellt werden. Die Zuckerlösung wiederum kann zur Herstellung von Biokraftstoffen oder Chemikalien etc. fermentiert werden. Gleichzeitig können die bei der enzymatischen Hydrolyse anfallenden Reststoffe zur Biogasgewinnung genutzt werden.

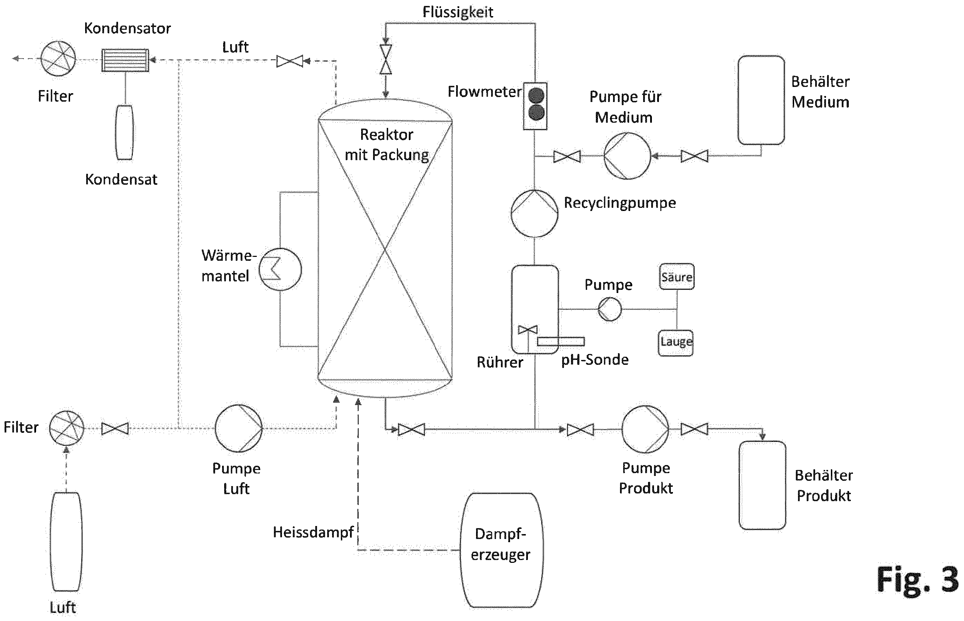

In Fig. 3 ist ein schematischer Aufbau eines Rieselstromreaktors gezeigt, mit dem die erfindungsgemäße Enzymproduktion mit den modifizierten filamentösen Pilzstämmen in dem Hydrolysat durchgeführt wird. Der Reaktor ist mit einem Kunststoffmaterial als Wachstumsoberfläche für die filamentösen Pilzstämme gefüllt. Das Hydrolysat zirkuliert von oben nach unten. Die Beaufschlagung mit Luft erfolgt von unten im Gegenstrom zur Flüssigkeit des Hydrolysats. Die Luft kann von oben abgezogen und in einem Kondensor kondensiert werden. Über eine pH-Sonde erfolgt die Überwachung des pH-Wertes. Dieser kann gegebenenfalls über die Dosierung von Säure und Lauge reguliert werden. Verschiedene Fermentationstechniken können durchgeführt werden. In einem Batch-Verfahren wird der Reaktor mit dem Medium befüllt. Das Hydrolysat wird während der Kultivierung im Kreis durch den Reaktor gepumpt. Darüber hinaus kann der Reaktor auch im kontinuierlichen Verfahren betrieben werden. Das Hydrolysat wird dabei kontinuierlich dem Prozess zugefügt. Ein Teil der Flüssigkeit wird dem Prozess allerdings auch wieder entzogen, so dass sich ein Gleichgewicht ausbildet.

Zur Vorbereitung des Fermentationsprozesses wird der Reaktor mit Heißdampf bei 121 °C für mindestens 25 min sterilisiert. Das aus der Vorbehandlung stammende Hydrolysat wurde mit einem Mineralmedium gemischt, welches folgende Zusammensetzung hat:

**Tabelle 1: Zusammensetzung des Mineralmediums**

| Komponente | Konzentration im Medium (g/l) |
|---|---|
| Natriumnitrat | 6 |
| Kaliumchlorid | 0,52 |
| Magnesiumsulfat | 0,52 |
| Kaliumdihydrogenphosphat | 1,52 |
| Spurenelemente* | 1 ml |

| | |
|---|---|
| Zusammensetzung der Spurenelemente: 2,2 g ZnSO₄, 7 H₂O, 1,1 g H₃BO₃, 0,5 g MnCl_{2',} * 4 H₂O, 0,5 g FeSO₄, 7 H₂O, 0,16 g CoCl₂ * 5 H₂O, 0,16 g CuSO₄ * 5 H₂O, 0,11 g Na₂MoO₄ * 4 H₂O und 5 g Na₂EDTA in 100 ml | |

Anschließend erfolgte eine Sterilisation des Mediums in einem Autoklav bei 121° C für wenigstens 20 min. Der pH wurde auf 6,5 eingestellt. Das modifizierte Hydrolysat enthält zusätzlich 1 mg/l Wachstumsfaktor p-Aminobenzoesäure oder Pyrodoxin. Das modifizierte Hydrolysat wurde dann in den Reaktor gefüllt. Die Animpfung des Reaktors erfolgte mit einer ausreichenden Menge an frischen Pilzsporen. Wenn alle Zucker verbraucht waren, wurde das Hydrolysat entfernt und der Reaktor mit einer sterilen Wasserlösung gespült, um das Wachstumsmedium zu entfernen. Nach dem Entfernen des Wassers wurde neues modifiziertes Hydrolysat als Produktionsmedium zu dem Reaktor zugegeben. Damit der Reaktor nicht zuwächst, war der Wachstumsfaktor in dem neuen Medium stark limitiert und enthielt lediglich 0,03 mg/l. Nachdem die Zucker wiederum verbraucht waren, wurde das alte Medium durch frisches Medium ersetzt und der Kreislauf von vorne gestartet.

Literatur:
Alvira, P., et al. (2010). "Pretreatment technologies for an efficient bioethanol production process based on enzymatic hydrolysis: A review." Bioresource technology 101(13): 4851-4861.
Chen, H., et al. (2017). "A review on the pretreatment of lignocellulose for high-value chemicals." Fuel Processing Technology 160: 196-206.
da Silva Delabona, P., et al. (2012). "Using Amazon forest fungi and agricultural residues as a strategy to produce cellulolytic enzymes." Biomass and bioenergy 37: 243-250.
Ghobadi, N., et al. (2017). "Characterizations of the submerged fermentation of Aspergillus oryzae using a Fullzone impeller in a stirred tank bioreactor." Journal of bioscience and bioengineering 123(1): 101-108.
Gottschalk, L. M. F., et al. (2010). "Cellulases, xylanases, β-glucosidase and ferulic acid esterase produced by Trichoderma and Aspergillus act synergistically in the hydrolysis of sugarcane bagasse." Biochemical Engineering Journal 51: 72-78.
Hall, M., et al. (2010). "Cellulose crystallinity-a key predictor of the enzymatic hydrolysis rate." Febs Journal 277(6): 1571-1582.
Howard, R., et al. (2004). "Lignocellulose biotechnology: issues of bioconversion and enzyme production." African Journal of Biotechnology 2(12): 602-619.
Jecu, L. (2000). "Solid state fermentation of agricultural wastes for endoglucanase production." Industrial Crops and Products 11(1): 1-5.
Jun, H., et al. (2011). "Enzyme production by filamentous fungi: analysis of the secretome of Trichoderma reesei grown on unconventional carbon source." Microbial Cell Factories 10(1): 68.
Kogo, T., et al. (2017). "Production of rice straw hydrolysis enzymes by the fungi Trichoderma reesei and Humicola insolens using rice straw as a carbon source." Bioresource Technology 233: 67-73.
Lee, H., et al. (2017). "Utilization of agricultural residues for enhancement of cellulolytic enzyme production and enzymatic saccharification by Trichoderma harzianum KUC1716." Industrial Crops and Products 109: 185-191.
Menon, V. and M. Rao (2012). "Trends in bioconversion of lignocellulose: biofuels, platform chemicals & biorefinery concept." Progress in Energy and Combustion Science 38(4): 522-550.
Müller, M., et al. (2015). "Continuous xylanase production with Aspergillus nudulans under pyridoxine limitation using a trickle bed reactor." Bioresource Technology 188:219-225 Sluiter, A., et al. (2008). "Determination of structural carbohydrates and lignin in biomass." National Renewable Energy Laboratorv(NREL/TP-510-42618).
Soccol, C. R., et al. (2017). "Recent developments and innovations in solid state fermentation." Biotechnology Research and Innovation.
WO 2017/177289 A1 (VTT INT OY [FI]), 19. Oktober 2017
Xu, J., et al. (2017). "Lactose-inducted production of a complete lignocellulolytic enzyme system by a novel bacterium Bacillus sp. BS-5 and its application for saccharification of alkali-pretreated corn cob." Cellulose 24(5): 2059-2070.
Zoglowek, M., et al. (2015). "Heterologous expression of cellobiohydrolases in filamentous fungi-An update on the current challenges, achievements and perspectives." Process Biochemistry 50(2): 211-220.

## Patentansprüche

1. Verfahren zur Herstellung von cellulolytischen Enzymen in Xylose-haltigen Hydrolysaten zum Abbau lignocellulosehaltiger Biomassen, umfassend die Schritte:
a) Bereitstellung eines genetisch modifizierten filamentösen Pilzstammes mit der Fähigkeit zur Überexpression von Cellobiohydrolasen, Endoglucanasen, Exocellulasen und/oder β-Glucosidasen in Xylose-haltigen Substraten als Produktionsmedium, deren natürliche Promotoren durch einen Xylanase-Promotor ersetzt worden sind,
b) Kultivierung des genetisch modifizierten filamentösen Pilzstammes unter nicht-limitierenden Bedingungen im Hydrolysat zur Bildung von Biomasse in einer ersten Phase,
c) Einstellen von wachstumslimitierenden Bedingungen in einer sich anschließenden zweiten Phase, sodass der Pilzstamm zwar die exprimierten cellulolytischen Enzyme produziert, ohne dabei selbst zu wachsen oder sich zu vermehren,
d) Isolierung der im Schritt c) produzierten cellulolytischen Enzyme, **dadurch gekennzeichnet, dass**
- die Kultivierung des genetisch modifizierten filamentösen Pilzsstammes unter wachstumslimitierenden Bedingungen in einem Rieselstromverfahren in einem Festbettreaktor erfolgt, wobei Festkörper mit Oberflächen zum Einsatz kommen, die von dem Pilzstamm bewachsbar sind, sodass das Pilzmyzel auf den Festkörpern wächst und dass ferner die Expression der cellulolytischen Enzyme durch Xylose induziert wird,
- es sich bei dem genetisch modifizierten filamentösen Pilzstamm um eine auxotrophe Mutante handelt, die zum Wachstum einen spezifischen Wachstumsfaktor benötigt,
- die wachstumslimitierenden Bedingungen durch Entzug des Wachstumsfaktors geschaffen werden und
- die Herstellung der cellulolytischen Enzyme durch eine Überexpression eines Transkriptionsaktivators verstärkt wird.

2. Verfahren nach Anspruch 1, **dadurch gekennzeichnet, dass** eine Expression der Cellobiohydrolasen, Endoglucanasen, Exocellulasen und/oder β-Glucosidasen durch den xynC Promotor erfolgt.

3. Verfahren nach Anspruch 2, **dadurch gekennzeichnet, dass** die Expression der cellulolytischen Enzyme über den xynC Promotor durch eine Überexpression des XInR-Transkriptionsaktivators verstärkt wird.

4. Verfahren nach Anspruch 1, **dadurch gekennzeichnet, dass** es sich bei dem Wachstumsfaktor um p-Aminobenzoesäure oder Pyridoxin handelt.

5. Verfahren nach einem der vorhergehenden Ansprüche, **dadurch gekennzeichnet, dass** es sich bei den genetisch modifizierten Pilzstämmen um *Aspergillus nidulans, Aspergillus niger, Aspergillus vadensis, Aspergillus feotidus, Aspergillus oryzae, Aspergillus aculeatus, Trichoderma reesei, Trichoderma harzianum, Neurospora crassa* handelt.

6. Verfahren nach einem der vorhergehenden Ansprüche, **dadurch gekennzeichnet, dass** die Bereitstellung der Xylose-haltigen Substrate für die Enzymproduktion durch eine thermisch-chemische Vorbehandlung von ligninhaltiger Biomasse mit einer verdünnten Säurelösung durchgeführt wird.

7. Verfahren nach einem der vorhergehenden Ansprüche, **dadurch gekennzeichnet, dass** eine Bestückung des Reaktors mit dem Hydrolysat von oben erfolgt.

8. Verfahren nach Anspruch 6, **dadurch gekennzeichnet, dass** von der Unterseite des Reaktors eine Zufuhr eines sauerstoffhaltigen Gasgemisches erfolgt.

9. Verfahren nach Anspruch 1, **dadurch gekennzeichnet, dass** dem Xylose-haltigen Hydrolysat vor der Kultivierung in Schritt b) ein Mineralmedium zugefügt wird.

10. Verfahren nach Anspruch 1, **dadurch gekennzeichnet, dass** es sich bei den Festkörpern um Kunststoffkörper handelt.

## Claims

1. Method for producing cellulolytic enzymes in xylose-containing hydrolysates for degradation of lignocellulose-containing biomasses, comprising the steps of:
a) providing a genetically modified filamentous fungal strain having the ability to overexpress cellobiohydrolases, endoglucanases, exocellulases and/or β-glucosidases in xylose-containing substrates as production medium, the natural promoters of which have been replaced by a xylanase promoter,
b) cultivating the genetically modified filamentous fungal strain under non-limiting conditions in the hydrolysate to form biomass in a first phase,
c) setting growth-limiting conditions in a subsequent second phase, so that the fungal strain produces the expressed cellulolytic enzymes without itself growing or propagating,
d) isolating the cellulolytic enzymes produced in step c), **characterized in that**
- the cultivation of the genetically modified filamentous fungal strain under growth-limiting conditions takes place in a trickle flow method in a fixedbed reactor, with use of solid bodies having surfaces which are coverable by the fungal strain, so that the fungal mycelium grows on the solid bodies and so that, furthermore, the expression of the cellulolytic enzymes is induced by xylose,
- the genetically modified filamentous fungal strain is an auxotrophic mutant which requires a specific growth factor for growth,
- the growth-limiting conditions are established by withdrawal of the growth factor, and
- the production of the cellulolytic enzymes is enhanced by overexpression of a transcription activator.

2. Method according to Claim 1, **characterized in that** the cellobiohydrolases, endoglucanases, exocellulases and/or β-glucosidases are expressed through the xynC promoter.

3. Method according to Claim 2, **characterized in that** the expression of the cellulolytic enzymes via the xynC promoter is enhanced by overexpression of the XInR transcription activator.

4. Method according to Claim 1, **characterized in that** the growth factor is p-aminobenzoic acid or pyridoxine.

5. Method according to any of the preceding claims, **characterized in that** the genetically modified fungal strains are *Aspergillus nidulans, Aspergillus niger, Aspergillus vadensis, Aspergillus feotidus, Aspergillus oryzae, Aspergillus aculeatus, Trichoderma reesei, Trichoderma harzianum, Neurospora crassa.*

6. Method according to any of the preceding claims, **characterized in that** the xylose-containing substrates for enzyme production are provided by thermal/chemical pre-treatment of lignin-containing biomass with a dilute acid solution.

7. Method according to any of the preceding claims, **characterized in that** the reactor is filled with the hydrolysate from above.

8. Method according to Claim 6, **characterized in that** an oxygen-containing gas mixture is supplied from the bottom side of the reactor.

9. Method according toClaim 1, **characterized in that** a mineral medium is added to the xylose-containing hydrolysate before the cultivation in step b).

10. Method according to Claim 1, **characterized in that** the solid bodies are plastics bodies.

## Revendications

1. Procédé de production d'enzymes cellulolytiques dans des hydrolysats contenant du xylose destiné à la dégradation de biomasses lignocellulosiques, comprenant les étapes consistant à :
a) fournir une souche fongique filamenteuse génétiquement modifiée capable de surexprimer des cellobiohydrolases, des endoglucanases, des exocellulases et/ou des β-glucosidases dans des substrats contenant du xylose en tant que milieu de production dont les promoteurs naturels ont été remplacés par un promoteur de xylanase ;
b) mettre en culture la souche fongique filamenteuse génétiquement modifiée dans des conditions non limitatives dans un hydrolysat pour une formation de biomasse dans une première phase ;
c) établir des conditions limitant la croissance dans une seconde phase ultérieure, de sorte que la souche fongique produise les enzymes cellulolytiques exprimées sans croître ni se multiplier ;
d) isoler des enzymes cellulolytiques produites à l'étape c), **caractérisé en ce que**
- la culture de la souche fongique filamenteuse génétiquement modifiée s'effectue dans des conditions limitant la croissance dans un réacteur à lit fixe selon un procédé à écoulement continu, en utilisant des corps solides ayant des surfaces qui peuvent être cultivées par la souche fongique, de sorte que le mycélium fongique se développe sur les corps solides et que l'expression des enzymes cellulolytiques est en outre induite par le xylose,
- la souche fongique filamenteuse génétiquement modifiée est un mutant auxotrophe qui a besoin d'un facteur de croissance spécifique pour se croître,
- les conditions limitant la croissance sont créées par le retrait du facteur de croissance, et
- la production des enzymes cellulolytiques est renforcée par une surexpression d'un activateur de transcription.

2. Procédé selon la revendication 1, **caractérisé en ce que** l'expression des cellobiohydrolases, des endoglucanases, des exocellulases et/ou des β-glucosidases survient via le promoteur xynC.

3. Procédé selon la revendication 2, **caractérisé en ce que** l'expression des enzymes cellulolytiques via le promoteur xynC est renforcée par une surexpression de l'activateur de transcription XInR.

4. Procédé selon la revendication 1, **caractérisé en ce que** le facteur de croissance est l'acide p-aminobenzoïque ou la pyridoxine.

5. Procédé selon l'une quelconque des revendications précédentes, **caractérisé en ce que** les souches fongiques génétiquement modifiées sont *Aspergillus nidulans, Aspergillus niger, Aspergillus vadensis, Aspergillus feotidus, Aspergillus oryzae, Aspergillus aculeatus, Trichoderma reesei, Trichoderma harzianum, Neurospora crassa.*

6. Procédé selon l'une quelconque des revendications précédentes, **caractérisé en ce que** la préparation des substrats contenant du xylose pour la production d'enzymes est effectuée par un prétraitement thermochimique de la biomasse contenant de la lignine avec une solution d'acide diluée.

7. Procédé selon l'une quelconque des revendications précédentes, **caractérisé en ce que** le réacteur est doté de l'hydrolysat par le haut.

8. Procédé selon la revendication 6, **caractérisé en ce qu'**une alimentation d'un mélange gazeux contenant de l'oxygène survient à partir de la face inférieure du réacteur.

9. Procédé selon la revendication 1, **caractérisé en ce qu'**un milieu minéral est ajouté à l'hydrolysat contenant du xylose avant la culture à l'étape b).

10. Procédé selon la revendication 1, **caractérisé en ce que** les corps solides sont des corps en matière plastique.
